(19) Europäisches Patentamt
European Patent Office
Office européen des brevets

(11) EP 2 448 570 B1

(12) **FASCICULE DE BREVET EUROPEEN**

(45) Date de publication et mention de la délivrance du brevet:
**16.11.2016 Bulletin 2016/46**

(21) Numéro de dépôt: **10731502.0**

(22) Date de dépôt: **01.07.2010**

(51) Int Cl.:
***A61K 31/198*** *(2006.01)* ***A61K 45/06*** *(2006.01)*
***A61P 17/00*** *(2006.01)* ***A61K 8/44*** *(2006.01)*

(86) Numéro de dépôt international:
**PCT/EP2010/059401**

(87) Numéro de publication internationale:
**WO 2011/000930 (06.01.2011 Gazette 2011/01)**

(54) **L-SERINE POUR SON UTILISATION EN TANT QUE MEDICAMENT POUR PREVENIR ET/OU TRAITER UNE REACTION INFLAMMATOIRE DE LA PEAU**

L-SERIN ALS WIRKSTOFF ZUR VERMEIDUNG UND/ODER BEHANDLUNG EINER ENTZÜNDUNGSREAKTION DER HAUT

L-SERINE TO BE USED AS A DRUG FOR PREVENTING AND/OR TREATING AN INFLAMMATORY RESPONSE OF THE SKIN

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **01.07.2009 FR 0954497**

(43) Date de publication de la demande:
**09.05.2012 Bulletin 2012/19**

(73) Titulaire: **Pierre Fabre Dermo-Cosmétique**
**92100 Boulogne-Billancourt (FR)**

(72) Inventeurs:
- **DECHELETTE, Corinne**
**F-81800 Rabastens (FR)**
- **CASTEX RIZZI, Nathalie**
**F-31770 Colomiers (FR)**
- **BONZOM, Laetitia**
**F-31130 Balma (FR)**

(74) Mandataire: **Regimbeau**
**20, rue de Chazelles**
**75847 Paris Cedex 17 (FR)**

(56) Documents cités:
**EP-A1- 1 762 241**
**EP-A1- 1 844 784**
**WO-A1-00/52051**
**WO-A1-2005/063266**
**WO-A1-2005/074910**
**WO-A1-2009/084200**
**WO-A2-2010/052312**
**JP-A- 11 209 285**
**JP-A- H08 217 695**
**US-A- 5 425 954**
**US-A1- 2003 008 018**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 448 570 B1

## Description

La présente invention se rapporte à l'utilisation de L-Sérine en tant que médicament pour prévenir et/ou traiter une réaction inflammatoire de la peau provoquée par une surexpression/suractivation des récepteurs PAR2, ainsi qu'en particulier à une composition dermo-cosmétique comprenant de la L-Sérine, de l'eau thermale d'Avène, de la glycérine, et un support cosmétiquement acceptable.

Le récepteur «Protease-Activated Receptor-2 (PAR-2) » est associé à la physiopathologie de plusieurs maladies impliquant des réponses inflammatoires (RATTENHOLL A et al ; Drug News Perspect ; 2008 Sep; 21(7):369-81).

Le PAR-2 appartient à la superfamille des récepteurs à 7 domaines transmembranaires couplés aux protéines G, mais possède une voie d'activation unique. En effet, le PAR-2 est activé par des Sérine-protéases telles que la trypsine, la tryptase, les facteurs Xa et VIIa. Le clivage par ces protéases de la partie extracellulaire du récepteur expose un nouveau domaine amino-terminal (SLIGKV) qui agit comme un ligand «attaché » au récepteur: il se fixe sur lui-même au niveau de la boucle extra-cellulaire 2 et s'auto-active.

Le PAR-2 est exprimé par les différents types cellulaires de la peau: kératinocytes, cellules myoépithéliales des glandes sudoripares, follicules pileux, cellules dentritiques-like du derme et les cellules endothéliales de la lamina propria et du derme (STEINHOFF et al., Exp Dermatol. ; 1999 Aug ; 8(4):282-94).

Les mélanocytes n'expriment pas ce récepteur bien que le PAR-2 joue un rôle important dans la pigmentation en favorisant le transfert de mélanine des mélanocytes vers les kératinocytes (SEIBERG et al., Exp Cell Res. ; 2000 Jan 10;254(1):25-32). Les Sérine-protéases générées par l'épiderme exercent des effets chimiotactiques induisant le recrutement des leucocytes dans la peau. Elles sont également impliquées dans la régulation de l'homéostasie, de la mitogenèse et de la différenciation épidermiques et modulent la fonction barrière de la peau. De plus, les Sérine-protéases contribuent à la physiopathologie de maladies cutanées liées à l'inflammation, la défense de l'hôte, la cancérogenèse, la fibrose et la stimulation nerveuse.

Les propriétés physiologiques et physiopathologiques cutanées des Sérine protéases seraient en partie liées aux récepteurs PARs. En effet, les récepteurs PAR-2 sont sur-exprimés dans l'épiderme, le derme et les vaisseaux des maladies inflammatoires de la peau comme la dermatite atopique, le lichen plan et le psoriasis (STEINHOFF et al., Exp Dermatol. ; 1999 Aug ;8(4):282-94).

Les récepteurs PAR-2 joueraient également un rôle dans le développement du prurit chez des patients atteints de dermatite atopique (STEINHOFF et al., J Neurosci. 2003 Jul 16; 23(15):6176-80).

L'activation de PAR-2 par une protéase de type trypsine induit la production d'IL-8 à partir de kératinocytes (HaCaT) (HOU et al., Immunology, 1998, 94:356-362). Chez les sujets présentant une peau sensible, il a été montré que les récepteurs PAR2 sont suractivés. La suractivation des PAR2 au niveau des fibres nerveuses sensitives a pour conséquence une sensation d'inconfort cutané. De plus, la suractivation des PAR2 au niveau des kératinocytes a pour conséquence une inflammation et restauration de la fonction barrière ralentie (sécrétion des corps lamellaires ralenties) (HACHEM JP et al, J Invest Dermatol. ; 2006 Sep;126(9):2074-86.).

Il existe donc un réel besoin de mettre au point des principes actifs permettant de diminuer l'expression et/ou l'activité des récepteurs PAR2.

De manière surprenante, la Demanderesse a mis en évidence que la L-Sérine, la L-Asparagine et/ou la L-Valine permettaient chacune d'inhiber l'expression/l'activation des récepteurs PAR2.

Ainsi, la présente invention se rapporte à de la L-Sérine pour son utilisation en tant que médicament pour prévenir et/ou traiter une réaction inflammatoire de la peau provoquée par une surexpression et/ou une suractivation des récepteurs PAR2 choisie parmi le prurit, caractérisée en ce que la L-sérine est le seul principe actif anti-prurit dudit médicament. Avantageusement, la présente description divulgue la L-Sérine pour son utilisation en tant que médicament pour prévenir et/ou traiter une réaction inflammatoire de la peau provoquée par une surexpression et/ou une suractivation des récepteurs PAR2. Avantageusement, la réaction inflammatoire de la peau provoquée par une surexpression et/ou une suractivation des récepteurs PAR2 est responsable d'une pathologie inflammatoire de la peau choisie dans le groupe constitué par le prurit..

Ainsi, avantageusement, la présente invention se rapporte à la L-Sérine pour son utilisation en tant que médicament pour prévenir et/ou traiter le prurit, caractérisée en ce que la L-sérine est le seul principe actif anti-prurit dudit médicament.

La L-Sérine est un acide-$\alpha$-aminé en C3, homologue hydroxylé de l'alanine.

La L-Sérine est un acide aminé aliphatique hydroxylé, dont le nom en nomenclature systématique est acide 2-amino-3hydroxypropanoïque de formule :

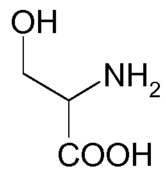

La L-Asparagine (ou acide asparagine-amino-succinique) est un acide α-aminé polaire non chargé et hydrophile, dérivé de l'acide aspartique présentant la formule suivante :

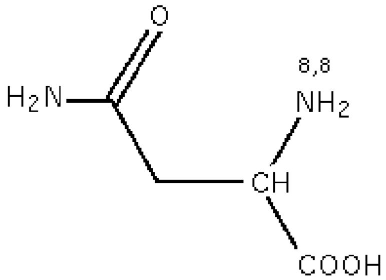

La L-valine est un acide α-aminé non polaire et hydrophobe, présentant la formule suivante :

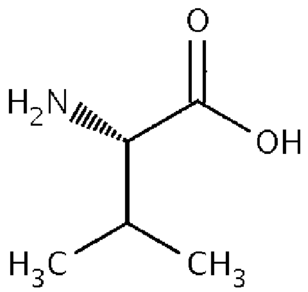

On entend par « surexpression des récepteurs PAR2 », le fait que lesdits récepteurs soient exprimés de façon plus importante que dans des conditions physiologiques normales.

On entend par « suractivation des récepteurs PAR2 », le fait que lesdits récepteurs présentent une activité plus importante que dans des conditions physiologiques normales.

Ainsi, la L-Sérine est utilisée pour inhiber les récepteurs PAR2.

On entend par « inhiber les récepteurs PAR2 », inhiber et/ou diminuer l'expression des récepteurs PAR2, ainsi qu'inhiber et/ou diminuer l'activité des récepteurs PAR2.

Au sens de la présente invention, les termes « prévenir » et « prévention » signifient éviter l'apparition d'une maladie, d'un trouble ou d'un ou plusieurs signes et/ou symptômes.

De façon préférée, la L-Sérine est utilisée en association avec de l'eau thermale d'Avène. La composition de l'eau thermale d'Avène est la suivante :

| Composition en mg /l | Avène |
|---|---|
| Chlorures | 5,4 |
| Bicarbonates | 226,7 |
| Hydrogénocarbonat es | |
| Sulfates | 13,1 |
| Silicates | |
| Silice | 14 |
| Nitrates | |

(suite)

| Composition en mg /l | Avène |
|---|---|
| Calcium | 42,7 |
| Magnésium | 21,2 |
| Sodium | 4,8 |
| Potassium | 0,8 |
| Fer | 0,005 |
| Sélénium | 0,005 |
| Silicium | |
| Zinc | 0,02 |
| Cuivre | 0,005 |
| Résidu sec | 207 |
| Minéralité | Faible |
| pH | 7,5 |
| Osmolarité | hypotonique |

Avantageusement, la L-Sérine est utilisée en association avec de la glycérine.
Encore plus avantageusement, la L-Sérine est utilisée en association avec de l'eau thermale d'Avène et de la glycérine.
On entend par glycérine ou glycérol, un propan-1,2,3-triol de formule :

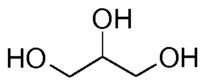

De préférence, la L-Sérine sera utilisée à une concentration supérieure à 30µM, de préférence à une concentration supérieure à 50µM, de manière encore préférée supérieure à 80µM, et de manière particulièrement préférée supérieure à 100 µM.
Selon un autre aspect, la présente invention se rapporte à une composition pharmaceutique comprenant de la L-Sérine pour son utilisation en tant que médicament pour prévenir et/ou traiter le prurit, caractérisée en ce que la L-sérine est le seul principe actif anti-prurit de la composition.
De façon préférée, la présente invention se rapporte à une composition pharmaceutique comprenant de la L-Sérine pour son utilisation en tant que médicament pour prévenir et/ou traiter le prurit, caractérisée en ce que la L-sérine est le seul principe actif anti-prurit de la composition.
De préférence, ladite composition est une composition dermatologique.
De manière particulièrement préférée, ladite composition est d'application topique.
On entend par « application topique » une application directement sur la peau.
Avantageusement, la composition pharmaceutique comprend en outre de l'eau thermale d'Avène et/ou de la glycérine, de préférence à l'exclusion de tout autre principe actif ayant un effet inflammatoire, et de manière particulièrement préférée à l'exclusion de tout autre principe actif.
De façon préférée, la L-Sérine est le seul principe actif de la composition ayant un effet anti-inflammatoire, et de manière particulièrement préférée est le seul principe actif.
Dans un mode particulier de réalisation la composition selon la présente divulgation peut en outre contenir des agents actifs destinés notamment à la prévention et/ou au traitement des affections cutanées.
De préférence, la composition selon la présente divulgation est utilisée pour son utilisation en tant que médicament pour prévenir et/ou traiter les peaux sensibles.
D'une manière générale, les peaux sensibles se définissent par une réactivité particulière de la peau.
Cette réactivité cutanée se traduit classiquement par la manifestation de signes d'inconfort en réponse à la mise en contact du sujet avec un élément déclenchant qui peut avoir diverses origines. Il peut s'agir de l'application d'un produit cosmétique en surface de la peau sensible, de la prise d'aliments, de l'exposition à des variations brutales de températures, à la pollution atmosphérique et/ou à des rayons aux ultra- violets ou infrarouges. Il existe également des facteurs associés comme l'âge et le type de peau. Ainsi les peaux sensibles sont plus fréquentes parmi les peaux sèches ou

grasses que parmi les peaux normales. Au sens de la présente invention, les peaux sensibles couvrent les peaux irritables et les peaux intolérantes.

Une peau intolérante est une peau qui réagit par des sensations d'échauffement, de tiraillements, de fourmillements et/ou de rougeurs, à différents facteurs tels que l'application de produits cosmétiques ou dermatologiques ou de savon. En général, ces signes sont associés à un érythème et à une peau hyper-séborrhéique ou acnéique, voire même rosacéiforme, avec ou sans dartres.

Une peau irritable est une peau qui réagit par un prurit, c'est-a- dire par des démangeaisons ou par des picotements, à différents facteurs tels que l'environnement, les émotions, les aliments, le vent, les frottements, le rasoir, l'eau dure à forte concentration de calcaire, les variations de température, l'humidité...

Avantageusement, la réaction inflammatoire de la peau provoquée par une surexpression et/ou une suractivation des récepteurs PAR2 est responsable d'une pathologie inflammatoire de la peau, ladite pathologie étant de préférence choisie dans le groupe constitué le prurit.

Ainsi, avantageusement, la présente invention se rapporte à une composition pharmaceutique comprenant de la L-Sérine pour son utilisation en tant que médicament pour prévenir et/ou traiter le prurit, caractérisée en ce que la L-sérine est le seul principe actif anti-prurit de la composition.

La dermatite atopique est décrite comme associée à un déficit dans le métabolisme des lipides du stratum corneum et notamment des céramides. Cette pathologie se présente sous la forme d'une xérose plus ou moins chronique concernant une grande étendue du corps, associée a des poussées inflammatoires et prurigineuses par plaques.

Le psoriasis est également une maladie inflammatoire cutanée d'évolution chronique qui affecte 2% de la population. Il est caractérisé par une croissance anormale des cellules épidermiques associée à une réaction inflammatoire.

Le lichen plan présente un aspect aléatoire - maladie auto immune - probablement liée au stress. Il présente un début brutal, faisant penser à une allergie : la peau se couvre de papules exémato-squameuses, qui démangent le patient. Le lichen plan peut être généralisé ou se cantonner à une petite surface de peau. Des douleurs musculaires, rhumatismales, peuvent accompagner les poussées de lichen plan. La maladie peut s'étendre de 12 à 15 mois.

La dermite séborrhéique est une dermatose touchant le visage et le cuir chevelu, caractérisée par des plaques rouges aux contours imprécis, avec des squames fines non adhérentes.

La rosacée est une maladie cutanée incurable au départ bénigne qui se manifeste par des rougeurs chroniques au niveau du nez, des joues, parfois aussi au niveau du menton et du front. Ces symptômes s'accompagnent d'une sensation de picotement, notamment au niveau des yeux.

La couperose est un état de rougeur permanente des zones convexes du visage (nez, joues, front, menton...), avec parfois de petits vaisseaux visibles à l'oeil nu.

De préférence, la composition selon la présente invention comprendra entre 0,01% et 10% en poids de L-Sérine par rapport au poids total de la composition et de préférence entre 0,5% et 3% de L-Sérine par rapport au poids total de la composition.

De façon préférée, la composition selon la présente invention comprendra entre 0,01% et 10% en poids de L-Sérine par rapport au poids total de la composition et de préférence entre 0,5% et 3% de L-Sérine par rapport au poids total de la composition.

La composition selon l'invention peut être formulée pour être administrée par voie topique, orale, sous cutanée, injectable, rectale ou génitale.

De préférence, la composition selon l'invention est formulée pour être administrée par voie topique.

Les compositions selon l'invention peuvent se présenter sous toutes les formes galéniques normalement utilisées selon le mode d'administration retenu.

Le support peut être de nature diverse selon le type de composition considérée.

En ce qui concerne plus particulièrement les compositions destinées à une administration par voie topique, il peut s'agir de solutions aqueuses, hydroalcooliques ou huileuses, de dispersions du type des solutions ou dispersions du type lotion ou sérum, d'émulsions de consistance liquide ou semi-liquide du type lait, de suspensions ou émulsions, du type crème, de gel aqueux ou anhydre, de microémulsions, de microcapsules, de microparticules, ou de dispersions vésiculaires de type ionique et/ou non ionique. Ces compositions sont préparées selon les méthodes usuelles.

Ces compositions peuvent notamment constituer des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des produits de maquillage comme des fonds de teint fluides, des laits de démaquillage, des laits corporels de protection ou de soin, des laits après-solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après- rasage, des crèmes épilatoires, ou des compositions contre les piqûres d'insectes.

Les compositions selon l'invention peuvent également consister en des préparations solides constituant des savons ou

des pains de nettoyage.

Elles peuvent être également utilisées pour les cheveux sous forme de solutions, de crèmes, de gels, d'émulsions, de mousses ou encore sous forme de compositions pour aérosol contenant également un agent propulseur sous pression.

Lorsque la composition de l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique et/ou dermatologique.

L'émulsionnant et le coémulsionnant peuvent être présents, dans la composition, en une proportion allant de 0,3 a 30 % en poids, et de préférence de 0,5 à 15% en poids par rapport au poids total de la composition.

Lorsque la composition de l'invention est un gel huileux, la phase grasse peut représenter plus de 90 % du poids total de la composition.

La composition cosmétique et/ou dermatologique de l'invention peut contenir également des adjuvants habituels dans le domaine cosmétique, pharmaceutique et/ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 à 35 % du poids total de la composition.

Ces adjuvants, selon leur nature, peuvent être introduits dans la phase grasse et/ou dans la phase aqueuse.

Comme matières grasses utilisables dans l'invention, on peut citer les huiles minérales comme par exemple le polyisobutène hydrogéné et l'huile de vaseline, les huiles végétales comme par exemple une fraction liquide du beurre de karité, huile de tournesol et d'amandes d'abricot, les huiles animales comme par exemple le perhydrosqualène, les huiles de synthèse notamment l'huile de Purcellin, le myristate d'isopropyle et le palmitate d'éthyl hexyle, et les huiles fluorées comme par exemple les perfluoropolyéthers. On peut aussi utiliser des alcools gras, des acides gras comme par exemple l'acide stéarique et comme par exemple des cires notamment de paraffine, carnauba et la cire d'abeilles. On peut aussi utiliser des composes siliconés comme les huiles siliconées et par exemple les cyclométhicone et diméthicone, les cires, les résines et les gommes siliconées.

Comme émulsionnants utilisables dans l'invention, on peut citer par exemple le stéarate de glycerol, le polysorbate 60, le mélange alcool cétylstéarylique/alcool cétylstéarylique oxyéthyléné à 33 moles d'oxyde d'éthylène vendu sous la dénomination Sinnowax AO® par la société HENKEL, le mélange de PEG-6/PEG-32/Glycol Stéarate vendu sous la dénomination de Tefose® 63 par société GATTEFOSSE, le PPG-3 myristyl ether, les émulsionnants siliconés tels que le cétyldiméthicone copolyol et le mono- ou tristéarate de sorbitane, le stéarate de PEG-40, le monostéarate de sorbitane oxyéthyléné (200E). Comme solvants utilisables dans l'invention, on peut citer les alcools inférieurs, notamment l'éthanol et l'isopropanol, le propylène glycol.

Comme gélifiants hydrophiles, on peut citer les polymères carboxyliques tel que le carbomer, les copolymères acryliques tels que les copolymères d'acrylates/alkylacrylates, les polyacrylamides et notamment le mélange de polyacrylamide, C13-14-Isoparaffine et Laureth-7 vendu sous le nom de Sepigel 305® par la société SEPPIC, les polysaccharides comme les dérivés cellulosiques tels que les hydroxyalkylcelluloses et en particulier les hydroxypropylcellulose et hydroxyéthylcellulose, les gommes naturelles telles que les guar, caroube et xanthane et les argiles.

Comme gélifiants lipophiles, on peut citer les argiles modifiées comme les bentones, les sels métalliques d'acides gras comme les stéarates d'aluminium et la silice hydrophobe, ou encore l'éthylcellulose et le polyéthylène.

Comme actifs hydrophiles, on peut utiliser les protéines ou les hydrolysats de protéine, les acides aminés, les polyols notamment en C2-C10 comme les glycerine, sorbitol, butylène glycol et polyéthylène glycol, l'urée, l'allantoïne, les sucres et les dérivés de sucre, les vitamines hydrosolubles, l'amidon, des extraits bactériens ou végétaux comme ceux d'Aloe Vera.

Comme actifs lipophiles, on peut utiliser le rétinol (vitamine A) et ses dérivés, le tocophérol (vitamine E) et ses dérivés, les céramides, les huiles essentielles et les insaponifiables (tocotriènol, sésamine, gamma oryzanol, phytostérols, squalènes, cires, terpènes).

Selon un autre aspect, la présente description divulgue l'utilisation cosmétique de la L-Sérine et/ou de la L-Asparagine et/ou de la L-Valine pour inhiber les récepteurs PAR2.

De façon préférée, la présente description divulgue l'utilisation cosmétique de la L-Sérine pour inhiber les récepteurs PAR2.

Selon un autre aspect, la présente description divulgue un procédé de traitement cosmétique et/ou pharmaceutique de prévention et/ou de traitement d'une réaction inflammatoire de la peau provoquée par une surexpression et/ou une suractivation des récepteurs PAR2, comprenant au moins une étape d'application de la composition pharmaceutique ou cosmétique de la présente description.

L'étape d'application peut par exemple être une application de crèmes, de gels, de sérums, de lotions, de laits de démaquillage ou de compositions après-solaires sur la peau ou sur les cheveux secs, application d'une lotion pour cheveux sur cheveux mouillés, de shampooings, ou encore application de dentifrice sur les gencives.

De préférence, ledit procédé est un procédé dermatologique, comprenant au moins une étape d'application

sur la peau d'une composition dermatologique telle que définie ci-avant. Le procédé cosmétique et/ou pharmaceutique divulgué par la description peut être mis en oeuvre par administration topique, journalière par exemple, de la composition selon l'invention.

Le procédé divulgué peut comprendre une étape d'application unique.

Selon un autre mode de réalisation, le procédé de traitement cosmétique comprend la répétition de l'étape d'application 2 à 3 fois par jour pendant un jour ou plus et généralement sur une durée prolongée d'au moins 4 semaines, voire 4 à 15 semaines, avec le cas échéant une ou plusieurs périodes d'interruption.

Selon un autre aspect, la présente invention se rapporte à une composition comprenant de la L-Sérine, de l'eau thermale d'Avène et de la glycérine, et un support pharmaceutiquement ou cosmétiquement acceptable.

De façon préférée, la présente invention se rapporte à une composition comprenant de la L-Sérine, de l'eau thermale d'Avène et de la glycérine, et un support pharmaceutiquement ou cosmétiquement acceptable.

Avantageusement, ladite composition est une composition dermo-cosmétique.

De préférence, ladite composition ne comprend aucun autre principe actif ayant un effet anti-inflammatoire, et de manière particulièrement préférée ne comprend aucun autre principe actif.

Exemple 1

Des modèles de kératinocytes de lignée HA Cat ont été utilisés. La L-Sérine et la L-Asparagine ont été achetées chez EVONIK, la L-Arginine a été achetée chez AMINO et la L-Valine a été achetée chez AJINOMOTO.

Une sonde fluorescente (Fluo-4/AM à une concentration de 2 μM) est incorporée pendant 30 minutes dans les cellules ensemencées dans des plaques 96 puits et l'actif à tester est incubé pendant 30 minutes. Seule la forme destérifiée de la sonde et liée aux ions calcium est excitable en fluorescence à 485 nm et émet à 535 nm. Le tampon d'incubation utilisé est un tampon HBSZS complémenté en HEPES (20mM) et en probénécide hydrosoluble (2,5mM).

Les cellules ont été stimulées avec de la trypsine à 10nM.

La molécule de référence utilisée est une molécule STI (Soybean Trypsin Inhibitor) à une concentration de 1μM.

La L-Sérine et la L-Asparagine, solubilisées à 50mg/mL dans de l'eau distillée (soit 475,8 mM pour la L-Sérine et 378, 4 mM pour la L-Asparagine) ont été évaluées aux concentrations de 3, 10, 30 et 100 μM.

La L-Arginine a été solubilisée à 100mM dans de l'eau distillée

La L-Valine a été solubilisée à 200mM dans de l'eau distillée.

Le L-Acide aspartique a été solubilisée à 200mM dans de l'eau distillée.

Le flux calcique, qui traduit une activation des récepteurs PAR2, est mesuré par fluorescence, puits par puits en temps réel selon une cinétique avant et après l'injection de trypsine. Le pourcentage d'inhibition d'activation du récepteur PAR2 correspond au pourcentage d'inhibition de flux calcique.

Les résultats présentés à la figure 1 montrent que l'application de L-Sérine permettait d'inhiber l'activation des récepteurs PAR2 de manière dose-dépendante.

Les résultats présentés dans le tableau 1 montrent que l'application de L-Asparagine permettait d'inhiber l'activation des récepteurs PAR2, mais pas de manière dose-dépendante.

Les résultats présentés dans le tableau 1 montrent que l'application de L-Valine permettait d'inhiber l'activation des récepteurs PAR2.

De plus, les résultats montrent que la L-Arginine ne présente aucune inhibition significative de l'activation des récepteurs PAR-2.

**Tableau 1**

| Pourcentage d'inhibition | Activité anti-PAR-2 (pourcentage d'inhibition) | | | |
|---|---|---|---|---|
| Concentration | 3 μM | 10 μM | 30 μM | 100 μM |
| L-Sérine | 3% | 13% | 20% | 33*** |
| L- Asparagine | 23%* | 23%* | 22%* | 31%*** |
| L-Arginine | -17% | 12% | -11% | 1% |
| L-Valine | 6% | 26%* | 20% | 34%*** |
| STI 1 μM | 106-109%*** | | | |
| p<0,5 et *** p<0,001 valeurs significativement différentes des valeurs Trypsine (Test de Dunnett) | | | | |

Exemple 2 :

La fonction barrière de la peau permet d'assurer une protection vis à vis de l'environnement extérieur. Les kératinocytes de l'épiderme peuvent directement répondre à une large variété d'agents irritants ou allergènes et participer activement aux processus inflammatoires et immunitaires cutanés, en particulier à travers la synthèse et la sécrétion de cytokines pro-inflammatoires, médiateurs d'origine protéique.
La chemokine - Interleukine 8 - qui est exprimée par les kératinocytes et qui est fortement impliquée dans l'amplification de la réponse inflammatoire, et a été plus spécifiquement étudiée La principale fonction de la chemokine est de recruter et d'activer les polynucléaires neutrophiles, notamment en stimulant leur sécrétion de molécules pro-inflammatoires.
Le test réalisé a permis d'évaluer l'activité anti-inflammatoire de la L-Sérine sur le niveau de sécrétion d'IL-8, stimulé par la Trypsine, de kératinocytes humains issus d'une lignée (HaCaT).

- Principe du dosage de l'IL-8 : ELISA de type sandwich permettant la quantification de l'IL-8 secrétée dans les surnageants de culture.

L'IL-8 est capturée par un anticorps monoclonal fixé aux puits de la microplaque. Un second anticorps anti-IL-8 est ajouté. Cet anticorps est biotinylé, ce qui permet la fixation de la streptavidine couplée à l'activité peroxidase. L'ajout du substrat de la peroxidase permet de quantifier la concentration en IL-8 de chaque puits par mesure de l'absorbance à 450nm.

- Matériel et Méthodes

a) Outil cellulaire:

Une lignée cellulaire de kératinocytes humains HaCaT a été utilisée.

b) Matériel:

Une microplaque à 96 puits pour culture cellulaire et un lecteur de plaque avec injecteurs :

Mithras LB940TM (Berthold Technologies®) ont été utilisés

c) Réactifs :

La trypsine à 10nM a été utilisée comme agent de stimulation.
Des réactifs du kit human CXCL8/IL8 Duoset R&D system (Réf. DY208(E)) ont été utilisés.

d) Produit testé :

La L-Sérine, solubilisée à 475,8mM dans de l'eau distillée, a été évaluée aux concentrations de 3μM, 10μM, 30μM, 100μM, 300μM et 1mM.

e) Protocole :

La L-Sérine est incubée 1 heure à 37°C (tampon HBSS). L'agent de stimulation (Trypsine T03) est ensuite ajouté et incubé 24 heures à 37°C.
Les surnageants de culture sont récupérés, centrifugés à +4°C puis stockés à -20°C.
Les concentrations en IL-8 des échantillons sont calculées à partir d'une courbe étalon d'équation : DO = (Bmax*[IL-8])/(Kd+[IL-8])
Dans un deuxième temps, les pourcentages d'inhibition de la sécrétion d'IL-8 sont calculés selon la formule :

$$\%\ \text{Inhibition} = 100 - \left( \frac{\text{Moy [IL-8] (Principe Actif)} - \text{Moy [IL-8] (Témoin)}}{\text{Moy [IL-8] (Agent de stimulation)} - \text{Moy [IL-8] (Témoin)}} \times 100 \right)$$

RESULTATS

Les valeurs indiquées dans le tableau ci-dessous représentent l'inhibition de la L-Sérine sur le niveau de sécrétion d'IL-8 suite à une stimulation Trypsine des kératinocytes HaCaT.

Pourcentage d'inhibition du niveau de sécrétion d'IL-8 :

Expérimentations isolées : (n=2)

Tableau 2

| | % inhibition | | | |
|---|---|---|---|---|
| | L-Sérine | | | |
| Facteur d'induction de la sécrétion d'IL-8 par la trypsine à 10nM | 10μM | 30μM | 100μM | 1mM |
| X 2.6 par rapport aux cellules contrôles (production basale sans stimulation) | 47*** | 50*** | 66*** | 74*** |
| *** p<0,001 valeurs significativement différentes des valeurs Trypsine (Test de Dunett) | | | | |

CONCLUSION

In vitro, à l'échelle cellulaire, une stimulation pro-inflammatoire telle que l'activation du récepteur PAR-2 par la trypsine induit la sécrétion d'IL-8 par les kératinocytes.

La L-Sérine présente des inhibitions significatives de la sécrétion d'IL-8 par les kératinocytes suite à la stimulation du récepteur PAR-2 par la trypsine avec une inhibition moyenne de 74% à la concentration de 1mM.

Exemple 3 : Composition selon l'invention

| INCI désignation | Pourcentage | Fonction |
|---|---|---|
| Eau | QSP 100% | |
| | | |
| Disodium EDTA | 0.2 | Agent complexant |
| Phenoxyethanol-Parabens | 0.8 | Conservateurs |
| glycerine | 4 | humectant |
| | | |
| carbomere | 0.5 | Gélifiant |
| | | |
| Glycéryl stéarate | 4 | Emulsionnant, facteur de consistance |
| Cétéaryl isononanoate | 3 | Emollient |
| Diméthicone | 5 | Emollient |
| squalane | 5 | Emollient |
| Paraffinium Liquidium | 10 | Emollient |
| | | |
| L-sérine | 1 | Actif |
| | | |

(suite)

| INCI désignation | Pourcentage | Fonction |
|---|---|---|
| triéthano lamine | 0.30 | Ajusteur de pH |

## Revendications

1. L-Sérine pour son utilisation en tant que médicament pour prévenir et/ou traiter le prurit, **caractérisée en ce que** la L-Sérine est le seul principe actif anti prurit dudit médicament.

2. Composition pharmaceutique comprenant de la L-Sérine pour son utilisation en tant que médicament pour prévenir et/ou traiter le prurit, **caractérisée en ce que** la L-Sérine est le seul principe actif anti prurit de la composition.

3. Composition pour son utilisation selon la revendication 2, comprenant en outre de l'eau thermale d'Avène.

4. Composition pour son utilisation selon l'une quelconque des revendications 2 ou 3, comprenant en outre de la glycérine.

5. Composition pour son utilisation selon l'une quelconque des revendications 2 à 4, dans laquelle la concentration de L-Sérine est comprise entre 0,01% et 10% en poids par rapport au poids total de la composition et de préférence entre 0,5% et 3% en poids par rapport au poids total de la composition.

6. Composition dermo-cosmétique comprenant de la L-Sérine, de l'eau thermale d'Avène, de la glycérine et un support cosmétiquement acceptable.

7. Utilisation de la L-Sérine comme seul principe actif anti-prurit pour la fabrication d'un médicament destiné à prévenir ou traiter le prurit.

## Patentansprüche

1. L-Serin für seine Verwendung als Arzneimittel, um dem Pruritus vorzubeugen und/oder ihn zu behandeln, **dadurch gekennzeichnet, dass** das L-Serin der einzige Anti-Pruritus-Wirkstoff des Arzneimittels ist.

2. Pharmazeutische Zusammensetzung, umfassend L-Serin für seine Verwendung als Arzneimittel, um dem Pruritus vorzubeugen und/oder ihn zu behandeln, **dadurch gekennzeichnet, dass** das L-Serin der einzige Anti-Pruritus-Wirkstoff der Zusammensetzung ist.

3. Zusammensetzung für ihre Verwendung nach Anspruch 2, umfassend außerdem Avene-Thermalwasser.

4. Zusammensetzung für ihre Verwendung nach einem der Ansprüche 2 oder 3, umfassend außerdem Glyzerin.

5. Zusammensetzung für ihre Anwendung nach einem der Ansprüche 2 bis 4, wobei die Konzentration von L-Serin zwischen 0,01 Gew.-% und 10 Gew.-% mit Bezug auf das Gesamtgewicht der Zusammensetzung und vorzugsweise zwischen 0,5 Gew.-% und 3 Gew.-% mit Bezug auf das Gesamtgewicht der Zusammensetzung liegt.

6. Dermo-kosmetische Zusammensetzung, umfassend L-Serin, Avene-Thermalwasser, Glyzerin und einen kosmetisch annehmbaren Träger.

7. Verwendung von L-Serin als einzigen Anti-Pruritus-Wirkstoff zur Herstellung eines Arzneimittels, das dazu bestimmt ist, dem Pruritus vorzubeugen oder ihn zu behandeln.

## Claims

1. L-Serine for use as a medicinal product for preventing and/or treating pruritus, **characterised in that** L-Serine is

the only antipruritic active ingredient of said medicinal product.

2. Pharmaceutical composition comprising L-Serine for use as a medicinal product for preventing and/or treating pruritus, **characterised in that** L-Serine is the only antipruritic active ingredient of the composition.

3. Composition for use according to claim 2, further comprising Avene thermal spring water.

4. Composition for use according to any one of claims 2 or 3, further comprising glycerine.

5. Composition for use according to any one of claims 2 to 4, wherein the concentration of L-Serine is between 0.01% and 10% by weight with respect to the total weight of the composition and preferably between 0.5% and 3% by weight with respect to the total weight of the composition.

6. Dermocosmetic composition comprising L-Serine, Avene thermal spring water, glycerine and a cosmetically acceptable substrate.

7. Use of L-Serine as the sole antipruritic active ingredient for manufacturing a medicinal product intended to prevent or treat pruritus.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **RATTENHOLL A et al.** *Drug News Perspect,* Septembre 2008, vol. 21 (7), 369-81
- **STEINHOFF.** *Exp Dermatol.,* Août 1999, vol. 8 (4), 282-94
- **SEIBERG.** *Exp Cell Res.,* 10 Janvier 2000, vol. 254 (1), 25-32
- **STEINHOFF et al.** *Exp Dermatol.,* Août 1999, vol. 8 (4), 282-94
- **STEINHOFF et al.** *J Neurosci.,* 16 Juillet 2003, vol. 23 (15), 6176-80
- **HOU et al.** *Immunology,* 1998, vol. 94, 356-362
- **HACHEM JP et al.** *J Invest Dermatol.,* Septembre 2006, vol. 126 (9), 2074-86